# EUROPEAN PATENT APPLICATION

(11) **EP 4 567 337 A1**
(43) Date of publication of application: **11.06.2025**
(21) Application number: 23875089.7
(22) Date of filing: 12.09.2023
(51) Int. Cl.: F24F 8/80, F24F 8/108, F24F 8/22, F24F 13/20, F24F 13/08, A61L 9/18

(54) **AIR-PURIFYING DEVICE**

(30) Priority: 06.10.2022 KR 20220128140; 03.11.2022 KR 20220145562
(71) Applicant: Samsung Electronics Co., Ltd., Suwon-si, Gyeonggi-do 16677 (KR)
(72) Inventor: PARK, Changhyun, Suwon-si, Gyeonggi-do 16677 (KR); KIM, Juyoung, Suwon-si, Gyeonggi-do 16677 (KR); KIM, Nakhyun, Suwon-si, Gyeonggi-do 16677 (KR); KIM, Huiyoung, Suwon-si, Gyeonggi-do 16677 (KR); PARK, Juwan, Suwon-si, Gyeonggi-do 16677 (KR); JUNG, Changwoo, Suwon-si, Gyeonggi-do 16677 (KR); CHO, Seoyoung, Suwon-si, Gyeonggi-do 16677 (KR); CHUN, Sunghyun, Suwon-si, Gyeonggi-do 16677 (KR)
(74) Representative: Gulde & Partner
(86) International application number: PCT/KR2023/013663
(87) International publication number: WO 2024/076021

(57) **Abstract**

An air inside the second housing purification apparatus may include an air purification module (20), an air sterilization module (30), and a connection module (4) that connects the air purification module (20) and the air sterilization module (30) to each other to enable air to flow therebetween, wherein the connection module (4) may include a connection housing (5) that connects the first housing (210) and the second housing (310) to each other and forms an exterior thereof, a connection passage (41) provided to allow at least a portion of air discharged through the first outlet (212) to be introduced into the connection housing (5) and flow into the second passage (P2) through the second inlet (311), a lighting portion (6) including a light source (65) and a reflective surface (61), wherein the light source (65) is arranged to surround a periphery of the connection passage (41), and the reflective surface (61) is configured to reflect light irradiated by the light source (65) and irradiate the light to outside of the connection housing (5), and a partition wall structure (8) provided to block introduction of air between the lighting portion (6) and the connection passage (41).

## Description

### Technical Field

Embodiments relate to an air purification apparatus.

### Background Art

Air purifiers are devices used to remove pollutants from the air. Air purifiers may remove bacteria, viruses, mold, fine dust, and other chemicals, which cause bad odors, from breathing air.

An air purifier may include an inlet through which polluted air is sucked in, an outlet through which purified air is discharged, and a blower fan configured to cause air to flow.

### Disclosure of Invention

### Solution to Problem

According to an embodiment, an air purification apparatus may include a first housing including a first inlet and a first outlet.

According to an embodiment, the air purification apparatus may include a first passage extending inside the first housing from the first inlet to the first outlet.

According to an embodiment, the air purification apparatus may include a first fan provided in the first passage.

According to an embodiment, the air purification apparatus may include an air purification module including a dust collection filter provided in the first passage.

According to an embodiment, the air purification apparatus may include a second housing including a second inlet and a second outlet.

According to an embodiment, the air purification apparatus may include a second passage extending inside the second housing from the second inlet to the second outlet.

According to an embodiment, the air purification apparatus may include a second fan provided in the second passage.

According to an embodiment, the air purification apparatus may include an air sterilization module including an ultraviolet light source provided in the second passage and configured to irradiate ultraviolet rays.

According to an embodiment, the air purification apparatus may include a connection module that connects the air purification module and the air sterilization module to each other to enable air to flow therebetween.

According to an embodiment, the connection module of the air purification apparatus may include a connection housing that connects the first housing and the second housing to each other and forms an exterior thereof.

According to an embodiment, the air purification apparatus may include a connection passage provided to allow at least a portion of air discharged through the first outlet to be introduced into the connection housing and flow into the second passage through the second inlet.

According to an embodiment, the air purification apparatus may include a light source arranged to surround a periphery of the connection passage.

According to an embodiment, the air purification apparatus may include a lighting portion including a reflective surface configured to reflect light irradiated by the light source and irradiate the light to outside of the connection housing.

According to an embodiment, the air purification apparatus may include a partition wall structure provided to block introduction of air between the lighting portion and the connection passage.

### Brief Description of Drawings

FIG. 1 is a perspective view of an air purification apparatus according to an embodiment.
FIG. 2 is a cross-sectional side view of an air purification module according to an embodiment.
FIG. 3 is a cross-sectional side view of an air sterilization module of an air purification apparatus, according to an embodiment.
FIG. 4 is a perspective view of a fan guard according to an embodiment.
FIG. 5 is an exploded perspective view of an air purification apparatus according to an embodiment.
FIG. 6 is an enlarged cross-sectional view of a connection area between an air sterilization module and an air purification module of an air purification apparatus, according to an embodiment.
FIG. 7 is an exploded perspective view of a connection module of an air purification apparatus, according to an embodiment.
FIG. 8 is a cross-sectional view centered on a connection module in an air purification apparatus, according to an embodiment.
FIG. 9 is a perspective view of a first connection housing and a circuit board according to an embodiment.
FIG. 10 is a perspective view of a partition wall structure according to an embodiment.
FIG. 11 is a perspective view of a second connection housing according to an embodiment.
FIG. 12 illustrates a light path of a lighting portion and a lighting area according to an embodiment.

### Mode for the Invention

Hereinafter, an air purification apparatus according to various embodiments is described in detail with reference to the accompanying drawings. In the drawings below, the same reference numeral refers to the same component, and the size of each component in the drawings may be exaggerated for clarity and convenience of explanation. The terms first, second, etc. may be used to describe various components, but the components should not be limited by these terms. The terms are used only to distinguish one component from another.

The singular forms "a", "an", and "the" are intended to include the plural forms as well, unless the context clearly indicates otherwise. When a portion "includes" a component, another component may be further included, rather than excluding the existence of the other component, unless otherwise described. Also, the size or thickness of each component in the drawings may be exaggerated for clarity of explanation.

The term "the" and similar referential terms may refer to both the singular and plural forms.

Steps of a method may be performed in any suitable order unless there is an explicit statement that the steps should be performed in the order described. In addition, all exemplary terms (e.g., etc., ...or the like, or ...and the like) are merely intended to elaborate technical ideas and does not limit the scope of the rights claimed, unless otherwise specified by the claims.

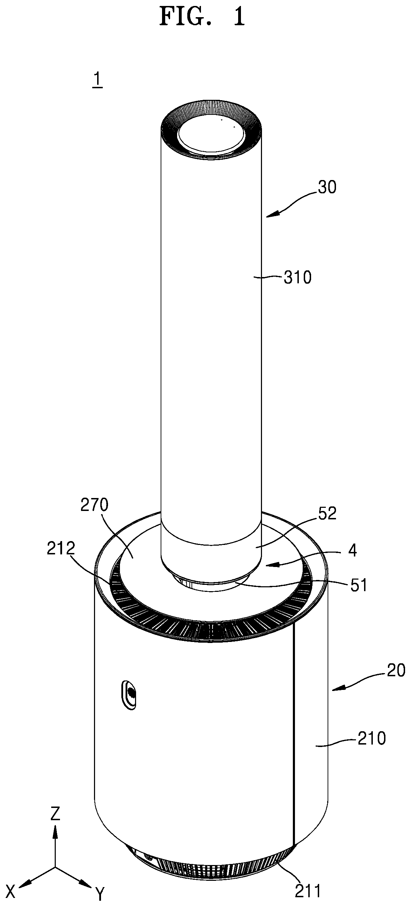

Moreover, the terms "vertical direction", "height direction", "direction perpendicular to...", etc. may refer to a Z-direction based on a perspective view of an air purification apparatus of FIG. 1, the term "horizontal direction" may refer to any direction along an X-Y plane in an X-direction or a Y-direction based on the perspective view of FIG. 1, and the shape and position of each component are not limited by these terms.

The terms "...or/er, portion, module, member, block", etc. used herein may be implemented as software or hardware, and according to embodiments, a plurality of "...ors/ers, portions, modules, members, or blocks may be implemented as a single component, or a single "...or/er, portion, module, member, or block" may include a plurality of components.

Hereinafter, an air purification apparatus according to an embodiment is described in detail with reference to the accompanying drawings.

FIG. 1 is a perspective view of an air purification apparatus according to an embodiment.

Referring to FIG. 1, the air purification apparatus according to an embodiment may include an air purification module 20 and an air sterilization module 30.

The air purification module 20 may purify air introduced from the outside and discharge purified air back to the outside. The air sterilization module 30 may sterilize air introduced from the outside and discharge the sterilized air back to the outside.

The air purification module 20 may be arranged to support the air sterilization module 30. The air purification module 20 may support the bottom of the air sterilization module 30. The air sterilization module 30 may be arranged over the air purification module 20. However, the disclosure is not limited thereto, and for example, the air purification module 20 and the air sterilization module 30 may each be provided such that lower portions thereof are supported on the ground.

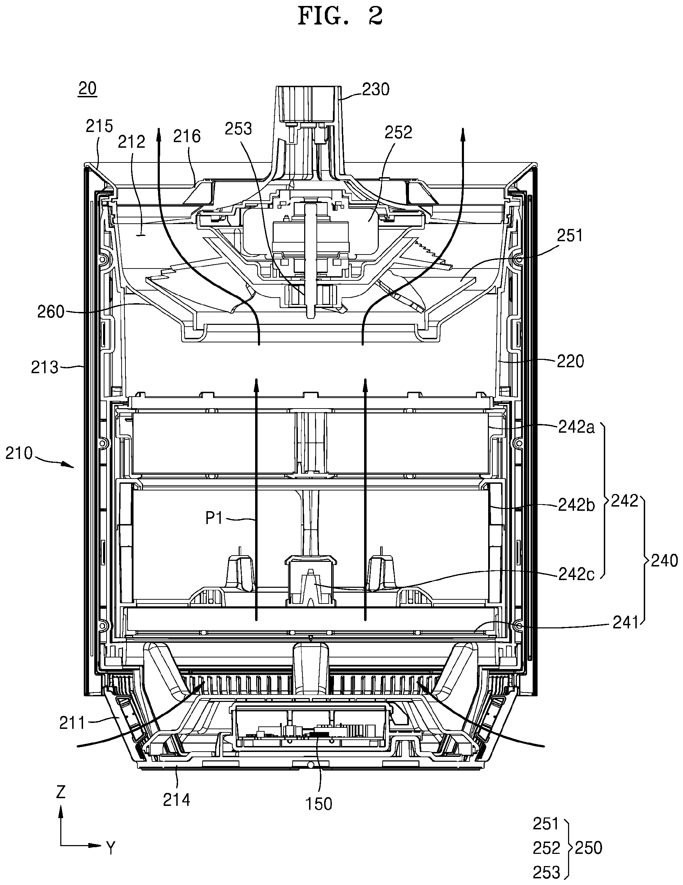

FIG. 2 is a cross-sectional side view of the air purification module 20 according to an embodiment.

Referring to FIG. 2, the air purification module 20 may include a first housing 210 in which a first passage P1 is provided. Various components of the air purification module 20 may be accommodated inside the first housing 210.

The first housing 210 may form an exterior of the air purification module 20. An exterior of the first housing 210 may have a cylindrical shape. However, the shape of the first housing 210 is not limited thereto and may vary.

A first inlet 211 may suck air outside the first housing 210 into the first housing 210. A first outlet 212 may discharge air inside the first housing 210 to the outside of the first housing 210. The air outside the first housing 210 may be sucked into the first housing 210 through the first inlet 211 due to pressure generated by the operation of a first fan 250 to be described below. The air inside the first housing 210 may be discharge to the outside of the first housing 210 through the first outlet 212 due to the pressure generated by the operation of the first fan 250.

The first passage P1 may extend from the first inlet 211 to the first outlet 212. The first fan 250 may discharge air through the first outlet 212 along the first passage P1, the air being introduced through the first inlet 211. The first inlet 211 may be provided at one end of the first passage P1, and the first outlet 212 may be provided at the other end of the first passage P1. The first inlet 211 may be formed below the first housing 210. The first outlet 212 may be formed over the first housing 210. The air introduced into the first housing 210 through the first inlet 211 may flow upward along the first passage P1.

However, the positions of the first inlet 211 and the first outlet 212 are not limited thereto, and the first inlet 211 and the first outlet 212 may be formed at various positions of the first housing 210.

The first housing 210 may include a first housing body 213, a base 214, a first upper cover 215, and a first outlet cover 216. The first housing body 213, the base 214, the first upper cover 215, and the first outlet cover 216 may each constitute a portion of the exterior of the air purification module 20.

The first housing body 213 may form horizontal sides of the air purification module 20. The first housing body 213 may cover various components of the air purification module 20 in a horizontal direction. The first housing body 213 may connect the base 214 and the first upper cover 215 to each other.

The first housing body 213 may cover the first passage P1 from the outside of the first passage P1 in a radial direction. The first passage P1 may be between the base 214 and the first outlet cover 216, and the first housing body 213 may cover the first passage P1 from the side of the first passage P1 in the horizontal direction.

The base 214 may be provided below the first housing 210. The base 214 may form the bottom surface of the air purification module 20. When the air purification module 20 is placed on the ground, the base 214 may support the air purification module 20. The base 214 may be coupled to the bottom of the first housing body 213.

The first inlet 211 may be formed in the base 214. The first inlet 211 may be arranged on the periphery of the base 214. The first inlet 211 may be arranged along the periphery of the bottom surface of the base 214. The side of the base 214 may be formed in a grill shape to allow air to enter and exit through the first inlet 211. However, the position at which the first inlet 211 is formed is not limited thereto, and the first inlet 211 may be formed at various positions of the air purification module 20.

The first upper cover 215 and the first outlet cover 216 may be provided over the first housing 210. The first upper cover 215 and the first outlet cover 216 may form upper surface of the air purification module 20. The first upper cover 215 and the first outlet cover 216 may be formed to cover an inner space of the first housing 210 from above. The first upper cover 215 may form a portion of the upper surface of the first housing 210, and the first outlet cover 216 may form another portion of the upper surface of the first housing 210.

The first outlet 212 may be formed on the upper surface of the first housing 210. The first outlet cover 216 may be provided to cover the first outlet 212.

The first outlet cover 216 may be formed in a grill shape to allow air to enter and exit through the first outlet 212. The grill shape of the first outlet cover 216 may be arranged along the periphery of the upper surface of the first housing 210. The grill shape of the first outlet cover 216 may be formed only on a portion of the upper surface of the first housing 210.

The first upper cover 215 may be arranged along the periphery of the first outlet cover 216. The first upper cover 215 may be coupled to the edge of the first outlet cover 216.

At least some of the first housing body 213, the base 214, the first upper cover 215, and the first outlet cover 216 may be integrally formed as a single body.

The air purification module 20 may include a first inner frame 220. The first inner frame 220 may be arranged inside the first housing 210. The first inner frame 220 may be coupled to an inner surface of the first housing 210 and support the inner surface of the first housing 210. The first inner frame 220 may support components of the air purification module 20.

A dust collection filter 240 and the first fan 250 may be arranged in an inner space of the first inner frame 220. The first passage P1 may be provided in the inner space of the first inner frame 220.

The air purification module 20 may include a first unit coupler 230 provided to be coupled to the air sterilization module 30. The first unit coupler 230 may be arranged over the air purification module 20.

The air purification module 20 may include the first fan 250. The first fan 250 may be provided in the first passage P1. The first fan 250 may be between the first inlet 211 and the first outlet 212. Air outside the air purification module 20 may be sucked in through the first inlet 211 due to a suction force of the first fan 250. The air sucked in through the first inlet 211 may flow along the first passage P1 and be discharged through the first outlet 212. The first fan 250 may include a first blade 251, a first motor 252, and a first fan rotation shaft 253 connected to the first motor 252 and configured to transmit, to the first blade 251, power generated by the first motor 252.

The first fan 250 may be between the dust collection filter 240 and the first outlet 212. When the first fan 250 is driven, the air sucked in through the first inlet 211 may sequentially pass through the dust collection filter 240 and the first fan 250 and be discharged through the first outlet 212.

The air purification module 20 may include a first fan support frame 260. The first fan 250 may be supported by the first fan support frame 260. The first fan support frame 260 may be supported by the first inner frame 220.

The air purification module 20 may include the dust collection filter 240. The dust collection filter 240 may be provided in the first passage P1. The dust collection filter 240 may collect foreign materials in the air sucked in through the first inlet 211. Foreign materials, such as organic materials including bacteria and viruses, or dust, present in air flowing along the first passage P1 may be removed from the air by the dust collection filter 240. The dust collection filter 240 may be formed such that an outer peripheral surface of the dust collection filter 240 is in contact with an inner peripheral surface of the first inner frame 220.

The dust collection filter 240 may be detachably mounted on the first inner frame 220. A user may separate or assemble the dust collection filter 240 from or into the first inner frame 220.

The dust collection filter 240 may include a pre-filter 241 and an electric dust collection filter 242. The pre-filter 241 may separate relatively large foreign materials from the air.

The electric dust collection filter 242 may collect foreign materials in the air by using an electrostatic force. The electric dust collection filter 242 may include a filter case 242a, a dust collector 242b, and a charging portion 242c. The filter case 242a may be supported by the first inner frame 220. The filter case 242a may support the dust collector 242b and the charging portion 242c. The charging portion 242c may charge foreign materials in the air. The dust collector 242b may collect the foreign materials charged by the charging portion 242c. Electrodes having different polarities may be connected to the dust collector 242b and the charging portion 242c.

The air purification apparatus may include a control apparatus 150 configured to control the operation of the air purification apparatus. The control apparatus 150 include an electronic component. The control apparatus 150 may control power supplied to the air purification apparatus.

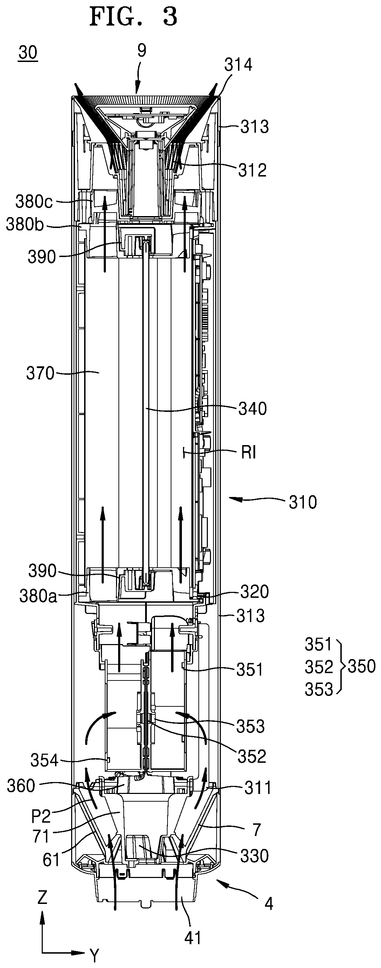

FIG. 3 is a cross-sectional side view of the air sterilization module 30 of an air purification apparatus, according to an embodiment.

Referring to FIG. 3, the air sterilization module 30 may include a second housing 310 in which a second passage P2 is provided. The second housing 310 may form an exterior of the air sterilization module 30. The second housing 310 may be configured to accommodate components of the air sterilization module 30 therein.

The second housing 310 may have a cylindrical shape. However, the shape of the second housing 310 is not limited thereto and may vary. A diameter of the second housing 310 may be less than a diameter of the first housing 210. In this case, a diameter of the air sterilization module 30 may be less than that of the air purification module 20. However, the diameter of the second housing 310 and the diameter of the first housing 210 are not limited thereto and may be equal to each other.

The second housing 310 of the air sterilization module 30 may include a second inlet 311 and a second outlet 312. The second inlet 311 may suck air outside the second housing 310 into the second housing 310. The second inlet 311 may suck, into the second housing 310, air introduced through a connection passage 41 from air outside the first housing 210 and the second housing 310 or air inside the first housing 210. The second outlet 312 may discharge air inside the second housing 310 to the outside of the second housing 310.

The air sterilization module 30 may include the second passage P2 between the second inlet 311 and the second outlet 312. The second passage P2 may extend from the second inlet 311 to the second outlet 312. A second fan 350 may discharge air through the second outlet 312 through the second passage P2, the air being introduced through the second inlet 311. The second inlet 311 may be provided at one end of the second passage P2, and the second outlet 312 may be provided at the other end of the second passage P2.

The second inlet 311 may be formed below the second housing 310. The second inlet 311 of the air sterilization module 30 may face the first outlet 212 of the air purification module 20. The second outlet 312 may be formed over the second housing 310. The second passage P2 may extend in a vertical direction. Air introduced into the second housing 310 through the second inlet 311 may flow upward along the second passage P2.

The second housing 310 may include a second housing body 313 and a second upper cover 314. The second housing body 313 and the second upper cover 314 may each constitute a portion of the exterior of the air sterilization module 30.

The second housing body 313 may form horizontal sides of the air sterilization module 30. The second housing body 313 may cover various components of the air sterilization module 30 in the horizontal direction. The second housing body 313 may be connected to the second upper cover 314. The second housing body 313 may be connected to a connection module 4.

The second housing body 313 may cover the second passage P2 from the outside of the second passage P2 in the radial direction. The second housing body 313 may cover the second passage P2 from the side of the second passage P2 in the horizontal direction. The second housing body 313 may extend in a direction parallel to the second passage P2. The second housing body 313 may extend in the vertical direction.

The second inlet 311 and the second outlet 312 may be formed in the second housing body 313. The second inlet 311 may be provided at one end of the second housing body 313 in a direction toward the connection module 4. The second outlet 312 may be provided at the other end of the second housing body 313, which is opposite to the second inlet 311. The second inlet 311 may be formed at the bottom of the second housing body 313. The second outlet 312 may be formed at the top of the second housing body 313.

The second upper cover 314 may be provided over the second housing 310. The second upper cover 314 may form the upper surface of the air sterilization module 30. The second upper cover 314 may cover an inner space of the second housing 310 from above.

The second upper cover 314 may cover at least a portion of the second outlet 312. The second upper cover 314 may guide the flow of air discharged through the second outlet 312. The second housing body 313 and the second upper cover 314 may be integrally formed as a single body.

The second outlet 312 may be positioned above the second passage P2. The second outlet 312 may have a grill shape to allow air to enter and exit. The second outlet 312 may discharge, to the outside of the second housing 310, air flowing through the second passage P2 inside the second housing 310.

The air sterilization module 30 may include a second inner frame 320. The second inner frame 320 may be arranged inside the second housing 310. The second inner frame 320 may be coupled to an inner surface of the second housing 310. The second inner frame 320 may support the inner surface of the second housing 310. The second inner frame 320 may support at least some of components of the air sterilization module 30. The second housing 310 may cover the second inner frame 320.

An ultraviolet light source 340 and a reflective cover 370 may be arranged in an inner space of the second inner frame 320. At least a portion of the second passage P2 may be provided in the inner space of the second inner frame 320.

The air sterilization module 30 may include the second fan 350. The second fan 350 may be provided in the second passage P2. The second fan 350 may be between the second inlet 311 and the second outlet 312. The second fan 350 may suck air outside the air sterilization module 30 into the second inlet 311. The air sucked in through the second inlet 311 may flow along the second passage P2 and be discharged through the second outlet 312.

The second fan 350 may suck air outside the second housing 310 into the second housing 310 through the second inlet 311. A fan guard 7 may be between the second fan 350 and the second inlet 311. The fan guard 7 may guide the flow of air moving to the second fan 350. The fan guard 7 may be arranged upstream of the second fan 350 in the second passage P2.

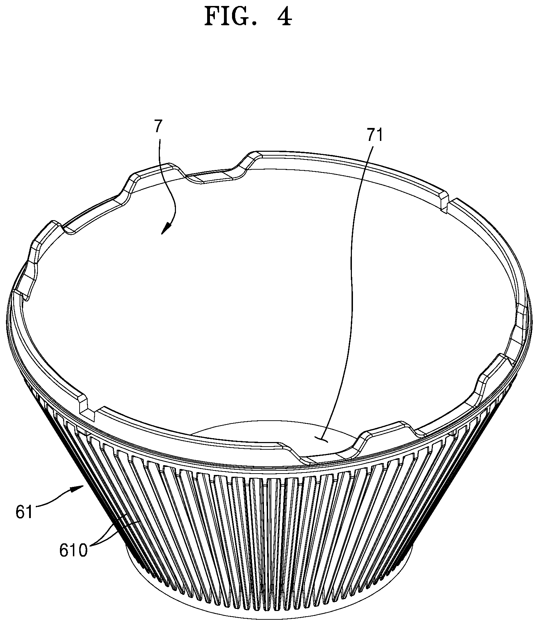

FIG. 4 is a perspective view of the fan guard 7 according to an embodiment.

Referring to FIGS. 3 and 4, the fan guard 7 may have a hollow cylindrical shape in which the diameter thereof increases upward. The fan guard 7 may have a substantially flare shape. However, the shape of the fan guard 7 is not limited thereto.

The fan guard 7 may include a fan guard cavity 71. The fan guard cavity 71 may be a space defined by an inner diameter of the fan guard 7. The top of the fan guard cavity 71 may have a larger diameter than the bottom of the fan guard cavity 71. The top of the fan guard cavity 71 may be connected to the second housing 310. The fan guard cavity 71 may define the second inlet 311.

Referring again to FIG. 3, the second fan 350 may discharge air inside the second housing 310 to the outside of the second housing 310 through the second outlet 312.

The second fan 350 may include a second blade 351, a second motor 352, and a second fan rotation shaft 353 connected to the second motor 352 and configured to transmit, to the second blade 351, power generated by the second motor 352. A plurality of second fans 350 may be arranged.

The second fan 350 may be between the ultraviolet light source 340 and the second inlet 311. When the second fan 350 is driven, air sucked in through the second inlet 311 may sequentially pass through the second fan 350 and the ultraviolet light source 340 and be discharged through the second outlet 312.

The air sterilization module 30 may include a second fan support frame 360. The second fan 350 may be supported by the second fan support frame 360. The second fan support frame 360 may be coupled to the second inner frame 320. The second fan support frame 360 may be coupled to the top of a second unit coupler 330.

The air sterilization module 30 may include the ultraviolet light source 340. The ultraviolet light source 340 may be provided in the second passage P2. The ultraviolet light source 340 may irradiate ultraviolet rays to air introduced through the second inlet 311. Organic materials, such as bacteria and viruses, present in air flowing along the second passage P2 may be removed from the air by the ultraviolet rays emitted from the ultraviolet light source 340. The ultraviolet light source 340 may sterilize the air flowing along the second passage P2.

The air sterilization module 30 may include the reflective cover 370 capable of reflecting the ultraviolet rays irradiated by the ultraviolet light source 340. The reflective cover 370 may cover the outside of the light source. The reflective cover 370 may be provided in the second passage P2.

An inner surface of the reflective cover 370 may include a material having high light reflectivity. The reflective cover 370 may surround the ultraviolet light source 340 from the sides of the light source 340. The reflective cover 370 may have a plurality of flat plate shapes, and the plurality of flat plate shapes may surround the ultraviolet light source 340 while forming a certain angle to each other. A plurality of reflective covers 370 may be provided.

The second passage P2 may include an irradiation area RI to which ultraviolet rays are irradiated by the ultraviolet light source 340. Air introduced into the second housing 310 through the second inlet 311 may be sterilized while passing through the irradiation area RI, such that organic materials, such as bacteria or viruses, may be removed.

The irradiation area RI may be provided inside the reflective cover 370. The irradiation area RI may be surrounded by the reflective cover 370. The reflective cover 370 may cover the irradiation area RI from the sides of the irradiation area RI in the horizontal direction.

The air sterilization module 30 may further include a light blocking member 380 provided to prevent ultraviolet rays from being incident on the outside of the irradiation area RI, the ultraviolet rays being irradiated by the ultraviolet light source 340. The light blocking member 380 may be provided in the second passage P2. The light blocking member 380 may prevent the ultraviolet rays from being emitted to the outside of the irradiation area RI.

The light blocking member 380 may include a first light blocking member 380a, a second light blocking member 380b, and a third light blocking member 380c. The first light blocking member 380a may be provided on one side of the irradiation area RI facing the second inlet 311. The second light blocking member 380b and the third light blocking member 380c may be arranged on the other side of the irradiation area RI facing the second outlet 312. A plurality of light blocking members 380 may be provided on the other side of the irradiation area RI facing the second outlet 312, thereby preventing the ultraviolet rays from being emitted to the outside of the air sterilization module 30 through the second outlet 312.

The air purification apparatus may include a display module 9. The display module 9 may include an input button and a display. The display module 9 may be arranged on the top of the air sterilization module 30.

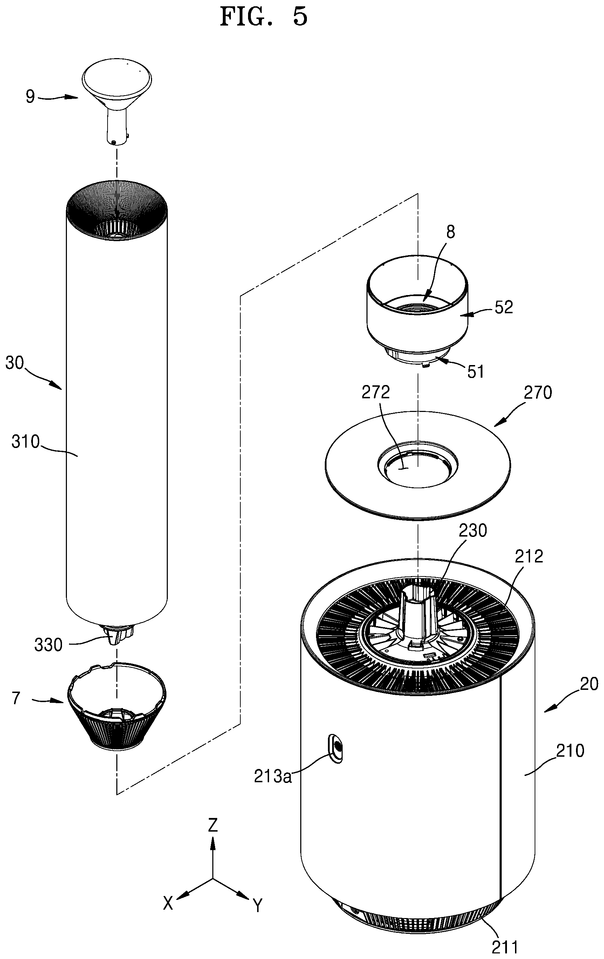

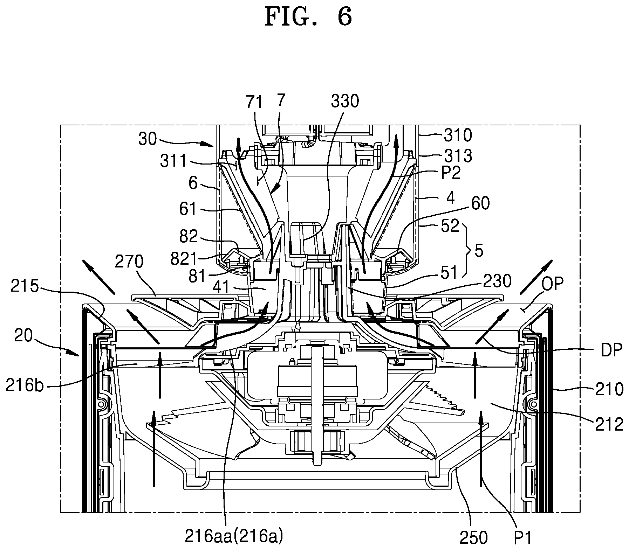

FIG. 5 is an exploded perspective view of the air purification apparatus according to an embodiment, and FIG. 6 is an enlarged cross-sectional view of a connection area between the air sterilization module 30 and the air purification mode 20 of the air purification apparatus, according to an embodiment.

Referring to FIGS. 5 and 6, in the air purification apparatus according to an embodiment, the air purification module 20 and the air sterilization module 30 may be coupled to each other. The first unit coupler 230 of the air purification module 20 may be coupled to the second unit coupler 330 of the air sterilization module 30. The second unit coupler 330 may have a protruding structure protruding toward the first unit coupler 230, and the first unit coupler 230 may have a groove structure into which the second unit coupler 330 may be inserted. A portion of the second unit coupler 330 may be assembled into the fan guard 7. However, the structures of the first unit coupler 230 and the second unit coupler 330 are not limited thereto and may be variously modified as long as the first unit coupler 230 and the second unit coupler 330 may be coupled to each other.

The air purification apparatus may include the connection module 4 that connects the air purification module 20 and the air sterilization module 30 to each other to enable air to flow therebetween.

The connection module 4 may include the connection passage 41. The connection passage 41 may connect the first passage P1 and the second passage P2 to each other to enable air to flow between the air purification module 20 and the air sterilization module 30. The connection passage 41 may be between the first passage P1 and the second passage P2.

The connection passage 41 may extend from at least a portion of the first outlet 212 to the first inlet 211. The connection passage 41 may be between at least a portion of the first outlet 212 and the second inlet 311. Air may be discharged without flowing from the first passage P1 to the connection passage 41. However, the flow of air from the first passage P1 to the connection passage 41 is not limited to the aforementioned description. For example, air flowing through the first passage P1 may all flow into the connection passage 41. For example, the air flowing through the first passage P1 may be discharged to the outside of the air purification module 20 through the first outlet 212 without flowing through the connection passage 41.

The connection module 4 may have a cylindrical shape. A diameter of the connection module 4 may be less than that of the air purification module 20. However, the shape of the connection module 4 is not limited thereto and may have various shapes as long as the shape allows the first passage P1 and the second passage P2 to be connected to each other.

At least a portion of the air discharged through the first outlet 212 may be introduced into the second inlet 311. The connection passage 41 may be between at least a portion of the first outlet 212 and the second inlet 311. The connection passage 41 may extend from at least a portion of the first outlet 212 to the second inlet 311. The air in the first passage P1 may be introduced into the second passage P2 along the connection passage 41. At least a portion of the air discharged through the first outlet 212 may be introduced into the connection passage 41 and flow into the second passage P2 through the second inlet 311.

The connection passage 41 may be arranged such that, when the second fan 350 is driven, the air in the first passage P1 may be introduced into the second passage P2. When the first fan 250 and the second fan 350 are simultaneously driven, the air introduced into the first passage P1 through the first inlet 211 may be introduced into the second passage P2 by the connection passage 41.

The connection passage 41 may extend in a direction from at least a portion of the first outlet 212 toward the second inlet 311. At least a portion of air discharged from the first housing 210 through the first outlet 212 may flow upward along the connection passage 41 and be introduced into the second housing 310 through the second inlet 311.

The first passage P1, the second passage P2, and the connection passage 41 may extend in a direction parallel to each other. The first passage P1, the second passage P2, and the connection passage 41 may each extend in a vertical direction of the air purification apparatus.

The air purification apparatus may include a discharge passage DP. The air in the first passage P1 discharged through the first outlet 212 may be discharged to the outside of the air purification apparatus through the discharge passage DP.

The air purification apparatus may include a discharge passage guide 270. The discharge passage guide 270 may guide the flow of air in the discharge passage DP. When the first fan 250 and the second fan 350 are simultaneously driven, the discharge passage guide 270 may guide at least a portion of the air discharged through the first outlet 212 to flow along the discharge passage DP, excluding another portion of the air introduced into the second passage P2 through the connection passage 41. The discharge passage guide 270 may cover at least a portion of the first outlet 212 from above.

The discharge passage DP may be formed by the discharge passage guide 270. At least a portion of the discharge passage DP may be covered by the discharge passage guide 270. The discharge passage DP may extend from at least a portion of the first outlet 212 toward a guide opening OP.

Outside air sucked in by the first inlet 211 of the air purification module 20 of the air purification apparatus may flow through the first passage P1 and pass through the first outlet 212, and then, a portion of the outside air may be discharged to the guide opening OP through the discharge passage DP. The outside air sucked in by the first inlet 211 of the air purification module 20 may flow through the first passage P1, a portion of the outside air may be discharged to the guide opening OP through the discharge passage DP, and another portion of the guide air may be sucked into the second inlet 311 through the connection passage 41 and flow into the second passage P2. However, the flow of the outside air sucked in by the first inlet 211 of the air purification module 20 is not limited to the aforementioned description.

A flow rate of air that may be efficiently processed by the air purification module 20 may differ from a flow rate of air that may be efficiently processed by the air sterilization module 30. When the flow rate of air purified by the air purification module 20 and the flow rate of air sterilized by the air sterilization module 30 are appropriately controlled, the air purification efficiency of the air purification apparatus may be increased.

According to an embodiment, a flow rate of the first passage P1 and a flow rate of the second passage P2 of the air purification apparatus may differ from each other. The flow rate of the first passage P1 may be greater than the flow rate of the second passage P2. The flow rate of air flowing through the second passage P2 may be a value obtained by subtracting a flow rate of air discharged through the discharge passage DP from the flow rate of air flowing through the first passage P1. However, the flow rates of air flowing through the first passage P1 and the second passage P2 are not limited to the aforementioned description.

The control apparatus 150 of the air purification apparatus may control a ratio of the flow rate of air flowing through the first passage P1 to the flow rate of air flowing through the second passage P2 in the air purification apparatus. The control apparatus 150 of the air purification apparatus may control power supplied to the first fan 250 and the second fan 350, thereby controlling the ratio of the flow rate of air flowing through the first passage P1 to the flow rate of air flowing through the second passage P2.

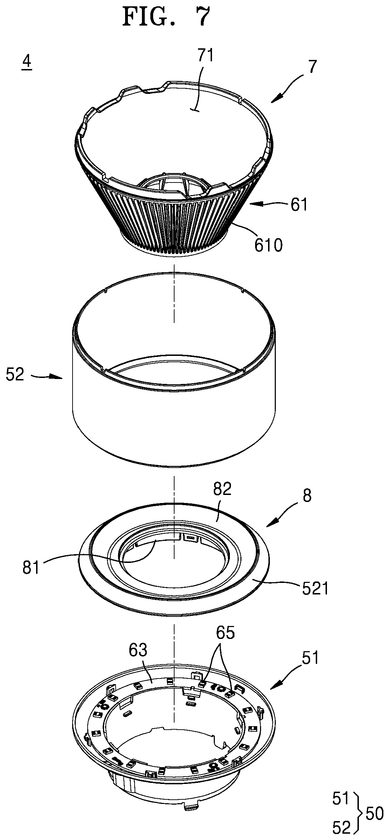

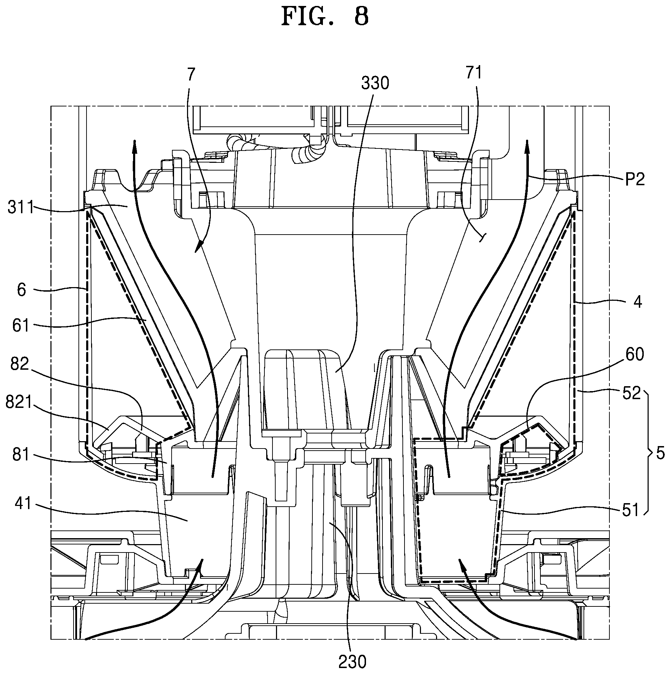

FIG. 7 is an exploded perspective view of the connection module 4 of the air purification apparatus, according to an embodiment. FIG. 8 is a cross-sectional view centered on the connection module 4 in the air purification apparatus, according to an embodiment.

Referring to FIGS. 6 to 8, the connection module 4 may include a connection housing 5 that forms an exterior thereof by connecting the first housing 210 and the second housing 310 to each other, and the connection passage 41 provided inside the connection housing 5. The connection housing 5 may include a first connection housing 51 and a second connection housing 52.

The first connection housing 51 may connect the first housing 210 and the second housing 310 to each other. The first connection housing 51 may be connected to the first housing 210. The bottom of the first connection housing 51 may be connected to the top of the first housing 210. The second connection housing 52 may be connected to the second housing 310. The top of the second connection housing 52 may be connected to the bottom of the second housing 310. The bottom of the first connection housing 51 may be connected to the first outlet cover 216. In this regard, connection does not only refer to direct connection but may also include indirect connection with another member therebetween. However, the connection relationship of the connection housing 5 is not limited to the aforementioned description.

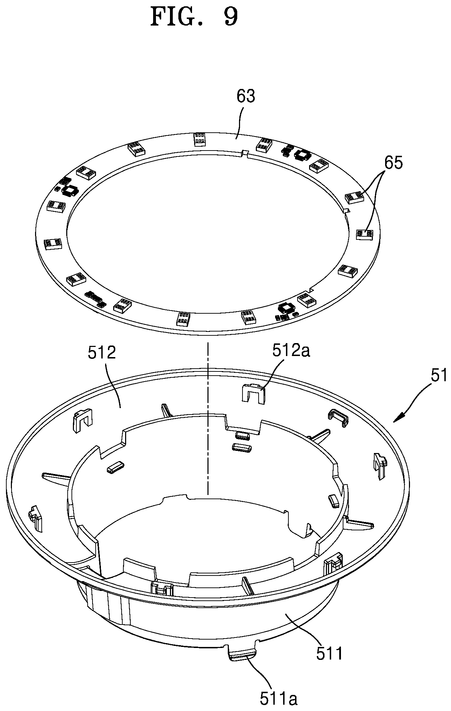

FIG. 9 is a perspective view of the first connection housing 51 and a circuit board 63 according to an embodiment.

Referring to FIGS. 7 to 9, the first connection housing 51 may include a connection passage housing 511 and a lighting housing 512.

The first connection housing 51 may define the connection passage 41. The connection passage housing 511 may define at least a portion of the connection passage 41. The connection passage 41 may connect the first passage P1 and the second passage P2 to each other in a space defined by the connection passage housing 511.

The connection passage housing 511 may have a shape in which a circular strip extends in the vertical direction. The connection passage housing 511 may have a hollow cylindrical shape. The connection passage housing 511 may have a substantially cylindrical shape. The connection passage housing 511 may have a shape in which the inside thereof is perforated. The bottom of the connection passage housing 511 may be connected to the first outlet cover 216. However, the shape of the connection passage housing 511 forming the first connection housing 51 is not limited thereto.

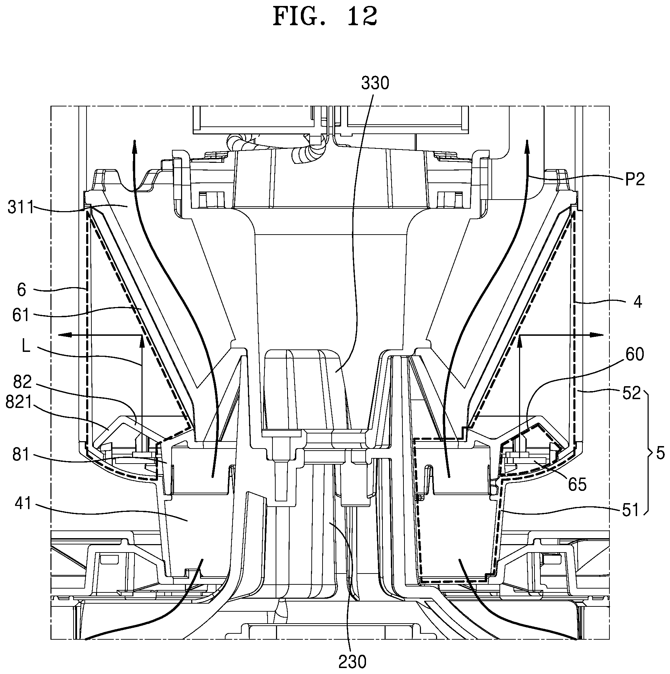

The lighting housing 512 may support the circuit board 63. The circuit board 63 may be disposed on the lighting housing 512. At least a portion of the lighting housing 512 may define a lighting area 60 (see FIG. 12) to be described below.

The lighting housing 512 may have a shape in which the lighting housing 512 branches off from the top of an outer peripheral surface of the connection passage housing 511 and the circumference of the top of the outer peripheral surface extends in a direction in which the diameter thereof increases. The lighting housing 512 may have a substantially plate shape in which the center there of is perforated. However, the shape of the lighting housing 512 forming the first connection housing 51 is not limited thereto.

One end of the lighting housing 512 may be connected to the upper outer peripheral surface of the connection passage housing 511. The other end of the lighting housing 512 may be elevated higher than the one end of the lighting housing 512 connected to the connection passage housing 511. The connection passage housing 511 and the lighting housing 512 may be formed as a single body. However, the shapes and connection relationship of the connection passage housing 511 and the lighting housing 512 are not limited to the aforementioned description.

The connection module 4 may include a lighting portion 6. The lighting portion 6 may surround the connection passage 41 in an annular shape. The lighting portion 6 may cover the connection passage 41 from the outside of the connection passage 41. However, the shape of the lighting portion 6 is not limited to the aforementioned description.

The lighting portion 6 may include a light source 65. The light source 65 included in the lighting portion 6 may include a light-emitting unit. For example, the light-emitting unit may include a point light source. A plurality of light-emitting units may be provided. However, the number and arrangement of light-emitting units are not limited thereto and may vary. For example, the light-emitting unit may include a linear light source. The light-emitting unit may include a single light source.

The lighting portion 6 may include the circuit board 63. The circuit board 63 may have a strip shape. The circuit board 63 may surround the connection passage 41 in an annular shape. However, the shape of the circuit board 63 is not limited thereto. The light source 65 may be disposed on the circuit board 63. A plurality of light sources 65 may be disposed radially at a certain interval on the circuit board. Elements other than the light source 65 may be mounted on the circuit board 63. The circuit board 63 may be disposed on the lighting housing 512.

The lighting portion 6 may include a reflective surface 61 configured to reflect light irradiated by the light source 65 and irradiate the light to the outside of the connection housing 5. The reflective surface 61 may be arranged on an outer peripheral surface of the fan guard 7.

The reflective surface 61 may include a material capable of reflecting light. The light source 65 of the lighting area 60 may irradiate light toward the fan guard 7. The reflective surface 61 may reflect light irradiated by the lighting area 60. The reflective surface 61 may reflect light to the outside of the second housing 310. The reflective surface 61 may reflect light to the outside of the second connection housing 52.

The reflective surface 61 may include a plurality of protrusions 610. The plurality of protrusions 610 may scatter light incident at a certain angle. The plurality of protrusions 610 may be arranged in parallel at a certain interval on the outer peripheral surface of the fan guard 7. However, the arrangement of the plurality of protrusions 610 is not limited to the aforementioned description. For example, the plurality of protrusions 610 may include any component that may be achieved by one of ordinary skill in the art through a design change as long as the component may scatter light incident on the reflective surface 61.

The first connection housing 51 may cover at least a portion of the first outlet 212. At least a portion of the air discharged through the first outlet 212 may flow into the first connection housing 51.

The first connection housing 51 may entirely cover the second inlet 311. At least a portion of the second inlet 311 may be covered. Air flowing along the first connection housing 51 may all flow into the second housing 310. At least a portion of the air flowing along the first connection housing 51 may be introduced into the second housing 310.

The first connection housing 51 may extend in a direction from at least a portion of the first outlet 212 toward the second inlet 311. At least a portion of the air discharged through the first outlet 212 may be introduced into the first connection housing 51, flow upward, and be introduced into the second housing 310 through the second inlet 311.

The first connection housing 51 may define the connection passage 41. The first connection housing 51 may define at least a portion of the connection passage 41. The connection passage housing 511 may define the connection passage 41. The connection passage housing 511 may define at least a portion of the connection passage 41. The connection passage 41 may be formed inside the first connection housing 51. The first connection housing 51 may cover the outside of the connection passage 41. However, the relationship between the first connection housing 51 and the connection passage 41 is not limited to the aforementioned description.

The first unit coupler 230 may be positioned at the center of the first connection housing 51. The second unit coupler 330 may be positioned at the center of the first connection housing 51. The first unit coupler 230 or the second unit coupler 330 may pass through the center of the first connection housing 51. The connection passage 41 may be between an inner peripheral surface of the first connection housing 51 and an outer peripheral surface of the first unit coupler 230. The connection passage 41 may be between the inner peripheral surface of the first connection housing 51 and an outer peripheral surface of the second unit coupler 330.

Air passing through the connection passage 41 may enter the fan guard cavity 71. The air discharged through the first outlet 212 may pass through the connection passage 41 of the first connection housing 51, enter the fan guard cavity 71 of the fan guard 7, and flow into the second passage P2. However, the flow of the air passing through the connection passage 41 is not limited to the aforementioned description.

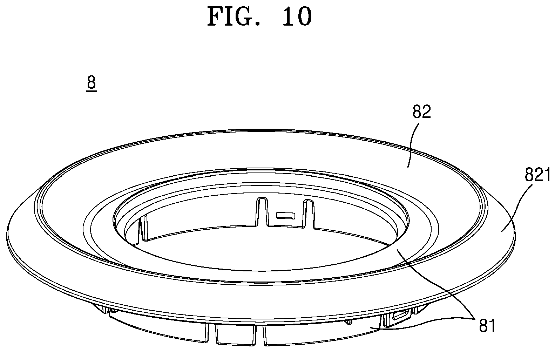

FIG. 10 is a perspective view of a partition wall structure 8 according to an embodiment.

Referring to FIGS. 7, 8, and 10, the connection module 4 may include the partition wall structure 8. The partition wall structure 8 may be provided to block the introduction or air between the lighting portion 6 and the connection passage 41. The partition wall structure 8 may prevent air from being introduced into the lighting portion 6. The partition wall structure 8 may prevent foreign materials included in the air from being introduced into the lighting portion 6. The partition wall structure 8 may prevent foreign materials from being introduced into the lighting portion 6. The partition wall structure 8 may be connected to the fan guard 7 and the first connection housing 51. The partition wall structure 8 may cover a space between the fan guard 7 and the first connection housing 51. The partition wall structure 8 may block air from flowing in between the fan guard 7 and the first connection housing 51. The partition wall structure 8 may guide the flow of air inside the connection passage 41. The partition wall structure 8 may cover the circuit board 63 on the first connection housing 51 from the top of the circuit board 63. The partition wall structure 8 may protect the circuit board 63. However, the arrangement and connection relationship of the partition wall structure 8 is not limited to the aforementioned description.

The lighting portion 6 may include the lighting area 60. The lighting area 60 may be an area defined by the partition wall structure 8 and the first connection housing 51. The circuit board 63 may be positioned inside the lighting area 60. The lighting area 60 may be an area where light irradiated by the light source 65 directly reaches. The lighting area 60 may irradiate light to the lighting portion 6.

The partition wall structure 8 may include a first partition wall 81 and a second partition wall 82.

The partition wall structure 8 may block air passing through the connection passage 41 from entering the lighting portion 6 of the connection module 4. The partition wall structure 8 may block air passing through the connection passage 41 from entering the lighting area 60. The first partition wall 81 may block air passing through the connection passage 41 from entering the lighting portion 6 of the connection module 4. The first partition wall 81 may block the introduction of air between the light source 65 of the lighting portion 6 and the connection passage 41.

When air does not enter the lighting portion 6, foreign materials, such as dust, that may be present in the air may not enter the lighting portion 6. When air does not enter the lighting area 60, foreign materials, such as dust, that may be present in the air may not enter the lighting area 60. When foreign materials, such as dust, do not enter the lighting portion 6, lighting quality may be improved. When foreign materials, such as dust, do not enter the lighting area 60, elements on the circuit board 63 may not be damaged.

The first partition wall 81 may be configured to block the introduction of air between the light source 65 and the connection passage 41. The first partition wall 81 may connect the fan guard 7 and the first connection housing 51 to each other and may block air passing through the connection passage 41 from being introduced between the fan guard 7 and the first connection housing 51. The top of the first partition wall 81 may be connected to the fan guard 7, and the bottom of the first partition wall 81 may be connected to the top of the connection passage housing 511. The first partition wall 81 may close a gap between the first partition wall 81 and the fan guard 7.

The first partition wall 81 may have a substantially cylindrical shape. The first partition wall 81 may have a hollow cylindrical shape and a shape in which the first partition wall 81 branches off from the top of a cylindrical inner peripheral surface and the top of the inner peripheral surface extends upward with a decreasing diameter. The first partition wall 81 may have a hollow cylindrical shape in which a neck thereof narrows approximately around a branch point. However, the shape of the first partition wall 81 is not limited thereto.

The second partition wall 82 may be between the fan guard 7 and the light source 65. An end of the second partition wall 82 may be connected to an end of the lighting housing 512. The second partition wall 82 may be between the light source 65 of the lighting area 60 and the fan guard 7. The second partition wall 82 may protect the lighting area 60.

The second partition wall 82 may be configured to allow light generated by the light source 65 to pass through at least a portion of the second partition wall 82. The second partition wall 82 may include a transparent material that allows light to pass therethrough. The second partition wall 82 may include a non-transparent film 821. The non-transparent film 821 may be arranged in a direction in which the diameter thereof increases from an intermediate end of the second partition wall 82. The non-transparent film 821 may be arranged in a direction away from the center at the top of the second partition wall 82. The non-transparent film 821 may prevent light irradiated by the light source 65 of the lighting area 60 from being irradiated to the outside without being reflected from the reflective surface 61.

The second partition wall 82 may be formed as a single body with the first partition wall 81. The second partition wall 82 may may include a shape in which the second partition wall 82 branches off from the top of a cylindrical outer peripheral surface of the first partition wall 81, extends upward with an increasing diameter, and then extends downward again with an increasing diameter. The second partition wall 82 may have a substantially disk shape in which the intermediate end thereof protrudes upward. However, the shape of the second partition wall 82 is not limited thereto.

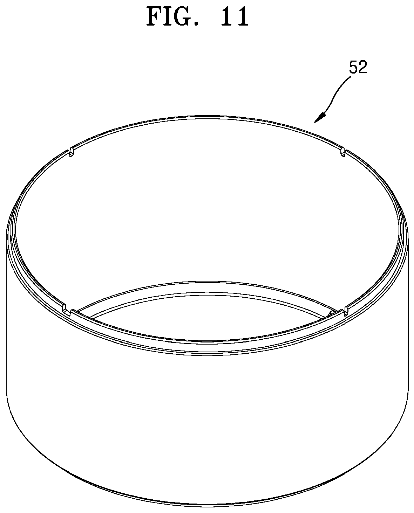

FIG. 11 is a perspective view of the second connection housing 52 according to an embodiment.

Referring to FIGS. 7, 8, and 11, the second housing 310 of the air sterilization module 30 may include the second connection housing 52.

The second connection housing 52 may be arranged to surround the fan guard 7. The second connection housing 52 may surround the second inlet 311. The top of the second connection housing 52 may be connected to the second housing 310. The bottom of the second connection housing 52 may be connected to the first connection housing 51. The bottom of the second connection housing 52 may be connected to the partition wall structure 8. The bottom of the second connection housing 52 may be connected to an end of the second partition wall 82.

The second connection housing 52 may have a shape in which a wall surface having a certain thickness extends in the vertical direction. The second connection housing 52 may have a cylindrical shape. The second connection housing 52 may have a substantially cylindrical shape. The second connection housing 52 may include a transparent material that allows light to pass therethrough. However, the shape of the second connection housing 52 is not limited thereto.

A cross-sectional shape of the second connection housing 52 may be the same as a cross-sectional shape of the second housing 310. A cross-sectional area of the second connection housing 52 may be the same as a cross-sectional area of the second housing 310. In this case, the cross-sectional area or cross-section may be a cross-sectional area or cross-section formed by an outer boundary of a figure when observed from the top and bottom.

FIG. 12 illustrates a light path of the lighting portion 6 and the lighting area 60 according to an embodiment.

Referring to FIG. 12, light L irradiated from the lighting area 60 of the lighting portion 6 may be reflected from the reflective surface 61 and emitted to the outside of the second connection housing 52. The light L irradiated from the lighting area 60 may pass through the second partition wall 82. The non-transparent film 821 of the second partition wall 82 may not transmit the light L. When the reflective surface 61 reflects the light L irradiated from the lighting area 60, the plurality of protrusions 610 may scatter the light L. The light L emitted to the outside of the second connection housing 52 may be light L reflected from the reflective surface 61. The light L emitted to the outside of the second connection housing 52 may be light L scattered by the plurality of protrusions 610.

The lighting portion 6 may provide indirect lighting. At least one of the second connection housing 52 and the second partition wall 82 may include a color filter. The air purification apparatus may serve as a mood light.

However, the arrangements and functions of the lighting portion 6 and the reflective surface 61 are not limited to the aforementioned description. For example, the light L emitted to the outside may be emitted to the outside without being reflected from the reflective surface 61. For example, the light L emitted to the outside may be emitted to the outside without passing through the second partition wall 82.

The aforementioned embodiments are merely examples, and those of ordinary skill in the art will appreciate that various modifications and other equivalent embodiments may be made from the aforementioned examples. Therefore, the scope of true technical protection according to the embodiments should be determined by the technical idea of the disclosure described in the following claims.

In an air purification apparatus according to an embodiment, a partition wall structure may block the inflow of air from a connection passage into a lighting portion. When the inflow of air into the lighting portion is blocked, dust may not accumulate in the light portion, thereby improving lighting quality.

According to an embodiment, an air purification apparatus may include an air purification module including a first housing, a first passage, a first fan, and a dust collection filter, wherein the first housing includes a first inlet and a first outlet, the first passage extends inside the first housing from the first inlet to the first outlet, the first fan is provided in the first passage, and the dust collection filter is provided in the first passage, an air sterilization module including a second housing, a second passage, a second fan, and an ultraviolet light source, wherein the second housing includes a second inlet and a second outlet, the second passage extends inside the second housing from the second inlet to the second outlet, the second fan is provided in the second passage, and the ultraviolet light source is provided in the second passage and configured to irradiate ultraviolet rays, and a connection module that connects the air purification module and the air sterilization module to each other to enable air to flow therebetween, wherein the connection module includes a connection housing that connects the first housing and the second housing to each other and forms an exterior thereof, a connection passage provided to allow at least a portion of air discharged through the first outlet to be introduced into the connection housing and flow into the second passage through the second inlet, a lighting portion including a light source and a reflective surface, wherein the light source is arranged to surround a periphery of the connection passage, and the reflective surface is configured to reflect light irradiated from the light source and irradiate the light to outside of the connection housing, and a partition wall structure provided to block introduction of air between the lighting portion and the connection passage.

The connection housing may include a first connection housing connected to the first housing and configured to define the connection passage, and a second connection housing connected to the second housing and surrounding the second inlet.

The air sterilization module may include a fan guard configured to define the second inlet and arranged upstream of the second fan in the second passage, and the second connection housing may be arranged to surround the fan guard.

The light source may be arranged to irradiate light toward the fan guard, the reflective surface may be arranged on an outer peripheral surface of the fan guard to reflect the light irradiated by the light source, and the second connection housing may be configured to allow the light reflected by the reflective surface to pass therethrough.

The reflective surface may include a plurality of protrusions.

A cross-sectional area of the second connection housing may be equal to a cross-sectional area of the second housing.

The lighting portion may surround the connection passage in an annular shape, and the second connection housing may have a cylindrical shape.

The partition wall structure may include a first partition wall configured to block introduction of air between the light source and the connection passage.

The first partition wall may connect the fan guard and the first connection housing to each other and may be configured to block air passing through the connection passage from being introduced between the fan guard and the first connection housing.

The partition wall structure may include a second partition wall between the fan guard and the light source.

The second partition wall may be configured to allow light generated by the light source to pass through at least a portion of the second partition wall.

The light source may include a plurality of light-emitting units arranged radially at a certain interval.

The air purification module, the air sterilization module, and the connection module may each have a cylindrical shape, and a diameter of the connection module may be less than a diameter of the air purification module.

The air sterilization module may be configured to suck in a portion of air discharged through the first outlet.

In the air purification apparatus, when the second fan is driven, a portion of air flowing along the first passage moves along the connection passage of the connection module and the second passage of the air sterilization module and is discharged through the second outlet.

## Claims

1. An air purification apparatus comprising:
an air purification module (20) comprising a first housing (210), a first passage (P1), a first fan (250), and a dust collection filter (240), wherein the first housing (210) comprises a first inlet (211) and a first outlet (212), the first passage (P1) extends inside the first housing (210) from the first inlet (211) to the first outlet (212), the first fan (250) is provided in the first passage (P1), and the dust collection filter (240) is provided in the first passage (P1);
an air sterilization module (30) comprising a second housing (310), a second passage (P2), a second fan (350), and an ultraviolet light source (340), wherein the second housing (310) comprises a second inlet (311) and a second outlet (312), the second passage (P2) extends inside the second housing (310) from the second inlet (311) to the second outlet (312), the second fan (350) is provided in the second passage (P2), and the ultraviolet light source (340) is provided in the second passage (P2) and configured to irradiate ultraviolet rays; and
a connection module (4) that connects the air purification module (20) and the air sterilization module (30) to each other to enable air to flow therebetween,
wherein the connection module (4) comprises:
a connection housing (5) that connects the first housing (210) and the second housing (310) to each other and forms an exterior thereof;
a connection passage (41) provided to allow at least a portion of air discharged through the first outlet (212) to be introduced into the connection housing (5) and flow into the second passage (P2) through the second inlet (311);
a lighting portion (6) comprising a light source (65) and a reflective surface (61), wherein the light source (65) is arranged to surround a periphery of the connection passage (41), and the reflective surface (61) is configured to reflect light irradiated by the light source and irradiate the light to outside of the connection housing (5); and
a partition wall structure (8) provided to block introduction of air between the lighting portion (6) and the connection passage (41).

2. The air purification apparatus of claim 1, wherein the connection housing (5) comprises:
a first connection housing (51) connected to the first housing (210) and configured to define the connection passage (41); and
a second connection housing (52) connected to the second housing (310) and surrounding the second inlet (311).

3. The air purification apparatus of claim 2, wherein the air sterilization module (30) comprises a fan guard (7) configured to define the second inlet (311) and arranged upstream of the second fan (350) in the second passage (P2), and the second connection housing (52) is arranged to surround the fan guard (7).

4. The air purification apparatus of claim 3, wherein the light source is arranged to
irradiate light toward the fan guard (7),
the reflective surface (61) is arranged on an outer peripheral surface of the fan guard 7 to reflect the light irradiated by the light source, and
the second connection housing (52) is configured to allow the light reflected by the reflective surface (61) to pass therethrough.

5. The air purification apparatus of claim 4, wherein the reflective surface (61) comprises a plurality of protrusions (610).

6. The air purification apparatus of claim 3, wherein a cross-sectional area of the second connection housing (52) is equal to a cross-sectional area of the second housing (310).

7. The air purification apparatus of claim 2, wherein the lighting portion (6) surrounds
the connection passage (41) in an annular shape, and
the second connection housing (52) has a cylindrical shape.

8. The air purification apparatus of claim 3, wherein the partition wall structure (8) comprises a first partition wall (81) configured to block introduction of air between the light source and the connection passage (41).

9. The air purification apparatus of claim 8, wherein the first partition wall (81) connects the fan guard (7) and the first connection housing (51) to each other and is configured to block air passing through the connection passage (41) from being introduced between the fan guard (7) and the first connection housing (51).

10. The air purification apparatus of claim 8, wherein the partition wall structure (8) comprises a second partition wall (82) between the fan guard (7) and the light source.

11. The air purification apparatus of claim 10, wherein the second partition wall (82) is configured to allow light generated by the light source to pass through at least a portion of the second partition wall (82).

12. The air purification apparatus of claim 1, wherein the light source comprises a plurality of light-emitting units arranged radially at a certain interval.

13. The air purification apparatus of claim 1, wherein the air purification module (20), the air sterilization module (30), and the connection module (4) each have a cylindrical shape, and
a diameter of the connection module (4) is less than a diameter of the air purification module (20).

14. The air purification apparatus of claim 1, wherein the air sterilization module (30) is configured to suck in a portion of air discharged through the first outlet (212).

15. The air purification apparatus of claim 14, wherein, when the second fan (350) is driven, a portion of air flowing along the first passage (P1) moves along the connection passage (41) of the connection module (4) and the second passage (P2) of the air sterilization module (30) and is discharged through the second outlet (312).
